# EUROPEAN PATENT APPLICATION

(11) **EP 2 829 269 A1**
(43) Date of publication of application: **28.01.2015**
(21) Application number: 14177753.2
(22) Date of filing: 21.07.2014
(51) Int. Cl.: A61K 31/192, A61K 9/00, A61K 9/20, A61P 29/00, A61P 19/02, A61P 19/04, A61K 47/00, A61K 47/32

(54) **Orally Disintegrating Tablet Formulation Comprising Flurbiprofen**

(30) Priority: 22.07.2013 TR 201308840
(71) Applicant: Sanovel Ilac Sanayi ve Ticaret A.S., 34460 Istanbul (TR)
(72) Inventor: Türkyilmaz, Ali, 34460 Istanbul (TR); Önder, Ramazan, 34460 Istanbul (TR); Pehlivan Akalin, Nur, 34460 Istanbul (TR)
(74) Representative: Sevinç, Erkan

(57) **Abstract**

The present invention relates to a formulation comprising flurbiprofen or a pharmaceutically acceptable salt of flurbiprofen. The present invention more particularly relates to an orally- disintegrating formulation of flurbiprofen, which is stable, does not have an unpleasant taste and is easily compressible.

## Description

### Field of Invention

The present invention relates to a formulation comprising flurbiprofen or a pharmaceutically acceptable salt of flurbiprofen. The present invention more particularly relates to an orally-disintegrating formulation of flurbiprofen, which is stable, does not have an unpleasant taste and is easily compressible.

### Background of Invention

Flurbiprofen is a propionic acid derivative, also known as NSAID (non-steroidal anti-inflammatory drug), having analgesic and anti-inflammatory activities. The chemical structure of flurbiprofen is illustrated in Formula I given below.

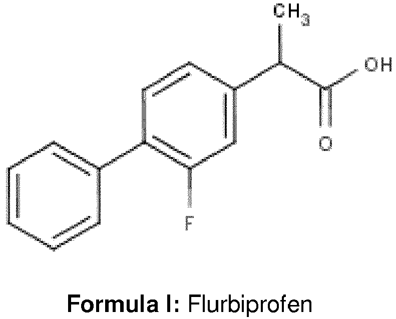

Flurbiprofen is used against mild to medium pains in the muscle-skeleton system and joint disorders such as ankylosing spondylitis, osteoarthritis, and rheumatoid arthritis, in soft tissue injuries such as sprains and strains, in postoperative pains, as well as dysmenorrhea and migraine. Flurbiprofen is further used as a lozenge in the symptomatic amelioration of sore throats.

The first desired effect of flurbiprofen when used in pain treatment is a rapid onset. Even if intravenous administration provides a rapid onset, it is disadvantageous since this is not a convenient form of administration. The effects of orally-disintegrating tablets take place much more rapidly as compared to solid oral dosage forms. Additionally, the use of flurbiprofen in treating local pains and inflammations may be problematic especially for those who have gastrointestinal system disorders. Various coating processes have been performed to prevent such side effects. The coatings, however, may cause problems in terms of solubility and thus bioavailability. Enhancing the absorption rate both provides ease of application and increases the molecular efficiency.

Besides the advantages of orally-disintegrating tablets, they are known to be difficult in terms of development. First, whilst an adequate oral disintegration is a desired feature, an early disintegration may be problematic typically based on the unpleasant taste of flurbiprofen. Particularly for orally-disintegrating formulations comprising a large amount of flurbiprofen, the unpleasant taste constitutes one of the major problems. Additionally, orally-disintegrating tablets should be quite porous and not too hard. Such porous tablets, however, are quite prone to be susceptible to humidity. As a result of this, they may show some stability problems.

In order to fulfill all these requirements described above, a special drug formulation should be adapted by a careful selection of the excipients to be used. The selected excipients, however, may give formulations with low bioavailability as compared to equivalent conventional dosage forms. For this reason, the excipients should be selected very carefully. In addition to these, measures should be taken while preparing, packaging, handling, and storing finished dosage forms of orally-disintegrating compositions, since they are prone to be hygroscopic and erosion.

Therefore, an orally-disintegrating tablet of flurbiprofen is needed, which would not leave a bitter taste in the mouth, has a porous structure, is well disintegrated in the mouth, and is easily punched or pressed during production. Other advantages and embodiments of the present invention will be clarified in the following description.

### Detailed Description of Invention

The main object of the present invention is to provide an orally-disintegrating tablet formulation comprising flurbiprofen or a pharmaceutically acceptable salt thereof which overcomes the problems mentioned above using suitable excipients.

Another object of the present invention is to provide an orally-disintegrating tablet formulation comprising a pharmaceutically effective amount of flurbiprofen and having a desired level of solubility and dissolution rate, and therefore a desired level of bioavailability.

A further object of the present invention is to provide an orally-disintegrating formulation of flurbiprofen or a pharmaceutically acceptable salt thereof, which has a suitable mechanical strength (e.g. an adequate hardness and low erosion) for not leaving a bitter taste in the mouth following disintegration, for processing in high-speed tablet presses, and for transporting in low-cost packages.

A further object of the present invention is to provide an orally-disintegrating tablet formulation comprising flurbiprofen or a pharmaceutically acceptable salt thereof which is stable and has a suitable bioavailability throughout the shelf life. Within the context of this object, unexpected advantages were found with an orally-disintegrating formulation according to the invention as compared to conventional solid dosage forms.

Mannitol is a sugar alcohol and is a frequently used excipient in pharmaceutical formulations. Even if mannitol with a high stability is used generally as a diluent in pharmaceutical formulations, it can be also used for different purposes according to the amounts and the types of mannitol. Since it is well-soluble in water, it leads to a rapid disintegration by destroying the tablet form in orally-disintegrating tablet formulations. Due to this feature, it can be used as a disintegrant in orally-disintegrating tablet formulations. Mannitol, which is not hygroscopic, can also be conveniently used with moisture-sensitive active ingredients. It prevents the oxidation of the equipment used during production. Since mannitol is a sugar alcohol, it is as sweet as glucose, and further aids in solving the taste problem of orally-disintegrating tablets. Mannitol, which also has a refreshing effect in the mouth, further contributes to the masking of the bitter taste originating from the active agent.

With this invention, a stable formulation having a high solubility and a high dissolution rate is surprisingly obtained. The formulation comprises flurbiprofen and mannitol having a mean particle size of 100 to 200 µm and granulated mannitol having a mean particle size of 300 to 400 µm. Preferably, mannitol having a mean particle size of 160 µm and granulated mannitol having a mean particle size of 360 µm are used. The formulation according to the present invention is obtained using wet granulation and the mannitol granules having the stated two different particle sizes are incorporated in different phases. Using mannitol having a mean particle size of 160 µm in the inner phase of the granules obtained via wet granulation in an amount of 30.00 to 40.00%, preferably 35.00 to 40.00% of the total tablet weight provided a rapid disintegration of the granules and leaves a refreshing effect in the mouth. On the other hand, using mannitol having a mean particle size of 360 µm in the outer phase of the granules obtained via wet granulation in an amount of 5.00 to 15.00%, preferably 8.00 to 12.00% of the total tablet weight aided in improving the flowability of the powder, and in preventing the problem in which the tablets stick to the punch during tablet pressing as caused by the mannitol granules having a mean particle size of 160 µm used in a higher ratio. While higher amounts can give rise to negative effects on the mechanical strength of the formulation, lower amounts may worsen the disintegration time. In result, using mannitol granules of the two different mean particle sizes given above makes it possible to substantially improve the compressibility of tablets, to reduce the erosion, to improve the taste, and to substantially reduce the disintegration time.

It was further surprisingly found that using crospovidone in an amount of 7.00 to 12.00% of the total tablet weight beside the mannitol granules having different mean particle sizes in the orally-disintegrating tablet formulation comprising flurbiprofen or a pharmaceutically acceptable salt thereof, and setting the ratio by weight of the mannitol granules having different mean particle sizes to crospovidone between 3:1 and 8:1 results in a synergistic effect on the disintegration time. The formulation according to the present invention disintegrates in the oral cavity in a time shorter than 90 seconds, preferably shorter than 40 seconds using the mannitol granules and crospovidone.

Since flurbiprofen is not dissolved in water in the orally-disintegrating tablets comprising flurbiprofen or a pharmaceutically acceptable salt thereof produced using wet granulation according to the prior art, the wet granulation process here was performed using water-soluble polymers. The water-soluble polymers used typically were polyvinyl alcohol (PVA), polyvinylpyrrolidone (PVP), or a mixture thereof. However, it was observed that the wet granules obtained via wet granulation using flurbiprofen molecules and polyvinyl alcohol, polyvinylpyrrolidone, or a mixture thereof collided with each other during the drying process and thus deteriorated the structure of the granules. It was further observed that flurbiprofen which turned back to a powder form stuck to the filters of the drier and thus resulted in a substantial loss of active agent. Using a higher amount of PVA, PVP, or a mixture thereof in an attempt of solving this problem, in turn, resulted in a prolongation of the oral disintegration time of the resulting orally-disintegrating tablets. However, it was surprisingly found that the problems described above did not occur when poly(butyl methacrylate-co-(2-dimetylaminoethyl)methacrylate-co-methyl methacrylate) 1:2:1 (Eudragit^{®} E PO) and stearic acid were used in the orally-disintegrating tablets of flurbiprofen produced using wet granulation. In orally-disintegrating tablets of flurbiprofen produced via wet granulation using poly(butyl methacrylate-co-(2-dimetylaminoethyl)methacrylate-co-methyl methacrylate) 1:2:1 in an amount of 2.00 to 8.00% of the total tablet weight and stearic acid in an amount of 0.01 to 1.00% of the total tablet weight, the active agent loss was notably reduced and the resulting tablet disintegrated in the oral cavity in a time shorter than 90 seconds. Additionally, since Eudragit^{®} E PO dissolves not in the oral cavity, but in the stomach with a lower pH, it does not make the oral disintegration of the orally-disintegrating tablet difficult, but it prevents the dissolution of the flurbiprofen molecules confined in the granules in the oral cavity. Thus, the bitter taste of flurbiprofen is masked in an excellent manner.

It was surprisingly found that using sodium stearyl fumarate as a lubricant in an amount of 0.50 to 3.00% of the total tablet weight provided a synergistic effect on the disintegration time, stability, and the production method. Sodium stearyl fumarate may fulfill several functions in the formulation according to the present invention. For instance, it may function both as a lubricant and a stabilizer. First, sodium stearyl fumarate is an extremely efficient lubricant and is less hydrophobic than magnesium stearate. It increases the wettability of the tablet ingredients so as to result in shorter disintegration times. Thus, in addition to the disintegrants, it assists in providing an easy oral disintegration of the orally-disintegrating tablet formulation. Additionally, sodium stearyl fumarate does not give rise to overmixing-related problems observed with magnesium stearate. Furthermore, sodium stearyl fumarate reduces friction and agglomeration better than magnesium stearate and thus allows for an easy compression of the tablets. Sodium stearyl fumarate, which can also be used as a stabilizer, assists in maintaining the stability of the tablet throughout the shelf life.

In another embodiment according to the present invention, colloidal silicon dioxide comprised in an amount of 0.01 to 0.50% of the total tablet weight assisted in improving the organoleptic properties of the tablet. Since colloidal silicon dioxide is composed of extremely fine silica particles, it keeps apart the powder particles from each other and thus reduces the attraction forces therebetween. Using colloidal silicon dioxide in an amount of 0.01 to 0.50% of the total tablet weight in the orally-disintegrating tablet formulation of flurbiprofen or a pharmaceutically acceptable salt thereof ensures the flowability required to obtain tablets having constant doses. It further prevents the agglomeration which is highly probable to take place during the filling process, and thus improves some properties of the tablet such as erosion and hardness.

Besides flurbiprofen, mannitol having two different mean particle sizes, crospovidone, poly(butyl methacrylate-co-(2-dimetylaminoethyl)methacrylate-co-methyl methacrylate) 1:2:1, stearic acid, colloidal silicon dioxide, and sodium stearyl fumarate, the orally-disintegrating tablets according to the present invention further comprises at least one pharmaceutically acceptable excipient selected from the group consisting of fillers, sweeteners, and aromatic agents.

Fillers suitable for use in the present invention include, but are not limited to sorbitol, sucrose, inorganic salts, calcium salts, microcrystalline cellulose, dextrose, dicalsium phosphate, sodium chloride, dextrates, lactitol, maltodextrin, sucrose-maltodextrin mixtures, xylitol, trehalose, heavy magnesium carbonate, or the mixtures thereof, wherein the filler is preferably microcrystalline cellulose. The orally-disintegrating tablet formulation according to the present invention comprises the filler in an amount of 1.00 to 5.0% of the total tablet weight.

Sweeteners suitable for use in the present invention include, but are not limited to sucralose, acesulfame-K, aspartame, sucrose, fructose, glucose, sorbitol, and the mixtures thereof, wherein the sweetener is preferably sucralose. The orally-disintegrating tablet formulation according to the present invention comprises the sweetener in an amount of 0.10 to 2.00% of the total tablet weight.

Aromatic agents suitable for use in the present invention include, but are not limited to fruit aromas such as orange, banana, strawberry, cherry, wild cherry, lemon; other aromas such as cardamom, anis, mint, menthol, vanillin, and ethyl vanillin or the mixtures thereof. The orally-disintegrating tablet formulation according to the present invention comprises the aromatic agent in an amount of 1.00 to 5.00% of the total tablet weight.

In order to minimize the disintegration time and prevent the bitter taste of the subject tablets, as well as to provide an easy compression thereof the orally-disintegrating tablet according to the present invention is designed to comprise the following ingredients:
a. 25.00 to 35.00% by weight of flurbiprofen,
b. 30.00 to 40.00% by weight of mannitol having a mean particle size of 160 µm,
c. 5.00 to 15.00% by weight of mannitol having a mean particle size of 360 µm,
d. 7.00 to 12.00% by weight of crospovidone,
e. 1.00 to 5.00% by weight of microcrystalline cellulose,
f. 0.10 to 2.00% by weight of sucralose,
g. 1.00 to 5.00% by weight of a mixture of mint and tutti-frutti aromas,
h. 2.00 to 8.00% by weight of Eudragit^{®} E PO,
i. 0.01 to 1.00% by weight of stearic acid,
j. 0.01 to 0.50% by weight of colloidal silicon dioxide,
k. 0.50 to 3.00% by weight of sodium stearyl fumarate.

The preferred method according to the present invention for preparing an orally-disintegrating tablet is wet granulation comprising the following steps:
a) introducing weighed amounts of flurbiprofen, microcrystalline cellulose, mannitol having a mean particle size of 160 µm, and the half amount of crospovidone into a granulator and mixing these ingredients,
b) mixing and dissolving water, poly(butyl methacrylate-co-(2-dimethylaminoethyl)methacrylate-co-methyl methacrylate) 1:2:1 and stearic acid in another solution container,
c) slowly introducing the solution prepared above to the powder mixture in the granulator and granulating these ingredients,
d) sieving the granules using a square 5-mm sieve and taking the granules into a fluidized bed dryer and drying the same,
e) passing the dried granules through a circular 0.8-mm sieve and grinding the granules,
f) then taking the granules to a container mixer and adding the remaining half of crospovidone, mannitol having a mean particle size of 360 µm, mint and tutti-frutti aromas, sucralose, and colloidal silicon dioxide to the container and mixing these ingredients,
g) adding sodium stearyl fumarate to the resulting mixture and mixing it until a homogeneous mixture is obtained and pressing each tablet in according with given specifications.

According to another aspect, the present invention shows that a significant influence can be achieved on the disintegration time of a tablet by changing the shape and the size thereof. In general, as the orally-disintegrating tablet becomes thinner and its porosity increases, it will become weaker more rapidly once it contacts with saliva, because the disintegration process takes place after the entire surface of the tablet is wetted by a capillary action. Additionally, any shape that will maximize the contact surface with saliva may significantly reduce the disintegration time.

The orally-disintegrating tablet composition of the present invention is preferably in the form of a disk, circle, sphere, hollow, bar, rectangle, polygon, ellipse, etc.. The preferred shape of the tablet is a rectangle.

The mean particle sizes of mannitol used in the orally-disintegrating formulation according to the present invention as an excipient were measured using a "dry method" according to the "laser diffraction" principle by means of a Malvern Particle Size instrument. According to the calculation principle of the instrument, the volume of the particles is converted to the volume of equivalent spheres, and the mean of the diameters of the spheres are given as a result. This particle size value is the volume moment mean D(4,3) value.

The orally-disintegrating formulation according to the present invention is for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.

The present invention is described in the following examples in more details. These examples are not limiting the scope of the present invention and should be considered under the light of the foregoing detailed disclosure.

### Examples

### Example 1: Orally-disintegrating tablet comprising flurbiprofen

| **Ingredients** | **Amount (%)** |
|---|---|
| Flurbiprofen | 25.00 - 35.00 |
| Microcrystalline cellulose PH 101 | 1.00 - 5.00 |
| Mannitol having a mean particle size of 160 µm (Pearlitol 160C^{®}) | 30.00 - 40.00 |
| Crospovidone | 7.00 - 12.00 |
| Mannitol having a mean particle size of 360 µm (Pearlitol 400DC^{®}) | 5.00 - 15.00 |
| Mixture of mint and tutti-frutti aromas | 1.00 - 5.00 |
| Sucralose | 0.01 - 2.00 |
| Eudargit^{®} E PO | 2.00 - 8.00 |
| Stearic acid | 0.01 - 1.00 |
| Colloidal silicon dioxide | 0.01 - 0.50 |
| Sodium stearil fumarat | 0.50 - 3.00 |

### Production method: Wet Granulation

Weighed amounts of flurbiprofen, microcrystalline cellulose PH (101), Pearlitol 160C^{®}, half of crospovidone are taken to a granulator and mixed for 10 minutes. In another solution container, water, Eudargit E PO are mixed and solved in stearic acid. The solution prepared above is slowly introduced to the powder mixture in the granulator and granulation is performed. The granules are sieved using a square 5-mm sieve and are taken to a fluidized bed dryer and dried there. The granules are dried until the humidity is 2.0% maximum. The dried granules are passed through a circular 0.8-mm sieve and ground. The granules are then taken to a container mixer and the remaining half of crospovidone, Pearlitol 400DC^{®}, the mixture of mint and tutti-frutti aromas, sucralose, and colloidal silicon dioxide are added to the granules in the container and mixed for 5 minutes. Then, sodium stearyl fumarate is added to this mixture and is stirred for around 3 more minutes. The tablets are pressed according to given specifications using the final homogeneous mixture.

### Example 2: Orally-disintegrating tablet comprising flurbiprofen

| **Ingredients** | **Amount (%)** |
|---|---|
| Flurbiprofen | 25.00 - 35.00 |
| Microcrystalline cellulose PH 101 | 1.00 - 5.00 |
| Mannitol having a mean particle size of 160 µm (Pearlitol 160C^{®}) | 35.00 - 40.00 |
| Crospovidone | 7.00 - 12.00 |
| Mannitol having a mean particle size of 360 µm (Pearlitol 400DC^{®}) | 8.00 - 12.00 |
| Mixture of mint and tutti-frutti aromas | 1.00 - 5.00 |
| Sucralose | 0.01 - 2.00 |
| Eudargit^{®} E PO | 2.00 - 8.00 |
| Stearic acid | 0.01 - 1.00 |
| Colloidal silicon dioxide | 0.01 - 0.50 |
| Sodium stearil fumarat | 0.50 - 3.00 |

This formulation is produced according to the production method described in Example 1.

## Claims

1. An orally-disintegrating tablet formulation comprising flurbiprofen or a pharmaceutically acceptable salt thereof, mannitol having a mean particle size of 100 to 200 µm and granulated mannitol having a mean particle size of 300 to 400 µm.

2. The orally-disintegrating tablet formulation according to claim 1, comprising mannitol having a mean particle size of 160 µm and granulated mannitol having a mean particle size of 360 µm.

3. The orally-disintegrating tablet formulation according to claim 2, wherein the amount of mannitol having a mean particle size of 160 µm is in range of 30.00 to 40.00% by weight of the total tablet formulation.

4. The orally-disintegrating tablet formulation according to claim 3, wherein the amount of mannitol having a mean particle size of 160 µm is in range of 35.00 to 40.00% by weight of the total tablet formulation.

5. The orally-disintegrating tablet formulation according to claim 2, wherein the amount of granulated mannitol having a mean particle size of 360 µm is in the range of 5.00 to 15.00% by weight of the total tablet formulation.

6. The orally-disintegrating tablet formulation according to claim 5, wherein the amount of granulated mannitol having a mean particle size of 360 µm is in the range of 8.00 to 12.00% by weight of the total tablet formulation.

7. The orally-disintegrating tablet formulation according to claims 1 to 6, further comprising crospovidone in an amount of 7.00 to 12.00% by weight of the total tablet formulation.

8. The orally-disintegrating tablet formulation according to claims 1 to 7, wherein the ratio of total weight of the mannitol ingredients having two different mean particle sizes to the total weight of crospovidone is between of 3:1 and 8:1.

9. The pharmaceutical formulation according to any of the preceding claims, further comprising poly(butyl methacrylate-co-(2-dimethylaminoethyl) methacrylate-co-methyl methacrylate) 1:2:1 in an amount of 2.00 to 8.00% by weight of the total formulation and stearic acid in an amount of 0.01 to 1.00% by weight of the total tablet formulation.

10. The pharmaceutical formulation according to any of the preceding claims, further comprising sodium stearyl fumarate in an amount of 0.50 to 3.00% by weight of the total tablet formulation.

11. The pharmaceutical formulation according to any of the preceding claims, further comprising colloidal silicon dioxide in an amount of 0.01 to 0.50% by weight of the total tablet formulation.

12. The pharmaceutical formulation according to any of the preceding claims, comprising the following ingredients:
a. 25.00 to 35.00% by weight of flurbiprofen,
b. 30.00 to 40.00% by weight of mannitol having a mean particle size of 160 µm,
c. 5.00 to 15.00% by weight of mannitol having a mean particle size of 360 µm,
d. 7.00 to 12.00% by weight of crospovidone,
e. 1.00 to 5.00% by weight of microcrystalline cellulose,
f. 0.10 to 2.00% by weight of sucralose,
g. 1.00 to 5.00% by weight of a mixture of mint and tutti-frutti aromas,
h. 2.00 to 8.00% by weight of poly(butyl methacrylate-co-(2-dimethylaminoethyl) methacrylate-co-methyl methacrylate) 1:2:1,
i. 0.01 to 1.00% by weight of stearic acid,
j. 0.01 to 0.50% by weight of colloidal silicon dioxide,
k. 0.50 to 3.00% by weight of sodium stearyl fumarate.

13. A process for preparing the pharmaceutical formulation according to any of the preceding claims, comprising the steps of
a) introducing weighed amounts of flurbiprofen, microcrystalline cellulose, mannitol having a mean particle size of 160 µm, and the half amount of crospovidone into a granulator and mixing these ingredients,
b) mixing and dissolving water, poly(butyl methacrylate-co-(2-dimethylaminoethyl)methacrylate-co-methyl methacrylate) 1:2:1 and stearic acid in another solution container,
c) slowly introducing the solution prepared above to the powder mixture in the granulator and performing granulation,
d) sieving the granules using a square 5-mm sieve and taking the granules to a fluidized bed dryer and drying the same,
e) passing the dried granules through a circular 0.8-mm sieve and grinding the granules,
f) then taking the granules to a container mixture and adding the remaining half of crospovidone, mannitol having a mean particle size of 360 µm, mint aroma, tutti-frutti aroma, sucralose, and colloidal silicon dioxide to the granules in the container and mixing these ingredients,
g) adding sodium stearyl fumarate to the resulting mixture and mixing it until a homogeneous mixture is obtained and pressing the mixture into tablets in accordance with given specifications.

14. The pharmaceutical formulation according to any of the preceding claims for use in mammalians and particularly in humans for the prevention or treatment of pain, arthralgia, toothache, myalgia, miosis inhibition, ankylosing spondylitis, osteoarthritis, rheumatoid arthritis and other muscle-skeleton system and joint disorders, soft tissue injuries such as sprains and strains, postoperative pains, painful and severe menstruation, migraine, and sore throat.
